# EUROPEAN PATENT SPECIFICATION

(11) **EP 2 604 686 B1**
(45) Date of publication and mention of the grant of the patent: **26.07.2017**
(21) Application number: 11816235.3
(22) Date of filing: 09.08.2011
(51) Int. Cl.: C12N 5/10, A61K 9/70, A61K 35/28, A61K 35/34, A61K 35/44, A61P 9/10, A61P 35/00, C12N 5/07, C12N 15/09, C12N 5/0775, C12N 5/074, C12N 9/22

(54) **HIGHLY FUNCTIONALIZED STEM CELL/PROGENITOR CELL BY APE1 GENE TRANSFECTION**
HOCHFUNKTIONALISIERTE STAMMZELLEN/VORLÄUFERZELLEN DURCH APE1-GENTRANSFEKTION
CELLULE SOUCHE/CELLULE PRÉCURSEUR À FONCTION ÉLEVÉE PAR INTRODUCTION DU GÈNE APE1

(30) Priority: 10.08.2010 JP 2010179605
(43) Date of publication of application: 19.06.2013
(73) Proprietor: National University Corporation Asahikawa Medical University, Hokkaido 078-8510 (JP)
(72) Inventor: KAWABE, Junichi, Asahikawa Hokkaido 078-8510 (JP); YAMAUCHI, Atsushi, Asahikawa Hokkaido 078-8510 (JP); TAKEHARA, Naofumi, Asahikawa Hokkaido 078-8510 (JP); HASEBE, Naoyuki, Asahikawa Hokkaido 078-8510 (JP)
(74) Representative: Hoffmann Eitle
(86) International application number: PCT/JP2011/004508
(87) International publication number: WO 2012/020566

(56) References cited:
- YAMAUCHI ATSUSHI ET AL: "Enhanced inhibition of Neointimal Hyperplasia by Overexpression of Apurinic/apyrimidinic Endonuclease (APE1) in Endothelial Progenitor Cells", CIRCULATION, vol. 122, no. 21, Suppl. S, November 2010 (2010-11), page A14862, XP002717917, & SCIENTIFIC SESSIONS OF THE AMERICAN-HEART-ASSOCIATION ON RESUSCITATION SCIENCE SYMPOSIUM; CHICAGO, IL, USA; NOVEMBER 13 -17, 2010
- HALL JENNIFER L ET AL: "Overexpression of Ref-1 inhibits hypoxia and tumor necrosis factor-induced endothelial cell apoptosis through nuclear factor-kappaB-independent and -dependent pathways", CIRCULATION RESEARCH, vol. 88, no. 12, 22 June 2001 (2001-06-22) , pages 1247-1253, XP002717911, ISSN: 0009-7330
- SONG Y J ET AL: "Tat-APE1/ref-1 protein inhibits TNF-alpha-induced endothelial cell activation", BIOCHEMICAL AND BIOPHYSICAL RESEARCH COMMUNICATIONS, ACADEMIC PRESS INC. ORLANDO, FL, US, vol. 368, no. 1, 28 March 2008 (2008-03-28), pages 68-73, XP027016414, ISSN: 0006-291X, DOI: 10.1016/J.BBRC.2008.01.037 [retrieved on 2008-01-17]
- HIBBERT BENJAMIN ET AL: "Inhibition of endothelial progenitor cell glycogen synthase kinase-3beta results in attenuated neointima formation and enhanced re-endothelialization after arterial injury.", CARDIOVASCULAR RESEARCH 1 JUL 2009, vol. 83, no. 1, 1 July 2009 (2009-07-01), pages 16-23, XP002717912, ISSN: 1755-3245
- YAMAUCHI ATSUSHI ET AL: "Apurinic/apyrimidinic endonucelase 1 maintains adhesion of endothelial progenitor cells and reduces neointima formation", AMERICAN JOURNAL OF PHYSIOLOGY - HEART AND CIRCULATORY PHYSIOLOGY, vol. 305, no. 8, October 2013 (2013-10), pages H1158-H1167, XP009175136,
- HEO J Y ET AL.: 'Downregulation of APE1/Ref-1 is involved in the Senescence of Mesencymal Stem Cells' STEM CELLS vol. 27, no. 6, 2009, pages 1455 - 1462, XP003031463
- CHENG C ET AL.: 'Protective effect of simvastatin on inflammatory injury of EPCs' JIANGSU MED J. vol. 35, no. 2, 2009, pages 205 - 207, XP008165908
- MOHRI T ET AL.: 'Signals Through Glycoprotein 130 Regulate the Endothelial Differentiation of Cardiac Stem Cells' ARTERIOSCLER THROMB VASC BIOL. vol. 29, 2009, pages 754 - 760, XP055070608
- ZOU GM ET AL.: 'The Ape-l/Ref-1 Redox Antagonist E3330 Inhibits the Growth of Tumor Endothelium and Endothelial Progenitor Cells: Therapeutic Implications in Tumor Angiogenesis' J CELL PHYSIOL. vol. 219, 2009, pages 209 - 218, XP055070637
- ZHANG Y ET AL.: 'Premature senescence of highly proliferative endothelial progenitor cells is induced by tumor necrosis factor-a via the p38 mitogen-activated protein kinase pathway' FASEB J. vol. 23, 2009, pages 1358 - 1365, XP005070640
- HENRICH D ET AL.: 'High Dosage of Simvastatin Reduces TNF-a-induced apoptosis of Endothelial Progenitor Cells but Fails to Prevent Apoptosis Induced by IL-lp In Vitro' J SURG RES. vol. 142, no. 1, 2007, pages 13 - 19, XP022208394

### SEQUENCE LISTING

<110> National University Corporation ASAHIKAWA MEDICAL UNIVERSITY
<120> High functional stem cell/precursor cell prepared by Ape1 gene transfection
<130> PAI-9003WO
<150> JP 2010-179605
   <151> 2010-08-10
<160> 5
<170> PatentIn version 3.51
   <210> 1
   <211> 22
   <212> DNA
   <213> Artificial
<220>
   <223> amplification primer for Ape1 (forward: mouse, rat)
<400> 1
   ggattgggta aaggaagaag ca 22
<210> 2
   <211> 22
   <212> DNA
   <213> Artificial
<220>
   <223> amplification primer for Ape1 (forward: human)
<400> 2
   gtgcccactc aaagtttctt ac 22
<210> 3
   <211> 22
   <212> DNA
   <213> Artificial
<220>
   <223> amplification primer for Ape1 (reverse: human, mouse, rat)
<400> 3
   caaggcgcca accaacattc tt 22
<210> 4
   <211> 21
   <212> DNA
   <213> Artificial
<220>
   <223> RT-PCR primer for Ape1 (forward: mouse)
<400> 4
   atgccgaagc gtgggaaaaa g 21
<210> 5
   <211> 19
   <212> DNA
   <213> Artificial
<220>
   <223> RT-PCR primer for Ape1 (reverse: mouse)
<400> 5
   cagtgctagg tatagggtg 19

## Description

### Technical Field

The present invention relates to the use of a highly functionalized stem cell/progenitor cell. More specifically, the present invention relates to the use of a highly functionalized stem cell/progenitor cell (e.g., endothelial progenitor cell or mesenchymal stem cell) improved in its functions such as angiogenesis by increased expression of Ape1.

### Background Art

Endothelial progenitor cells (EPCs) are a population of CD34-positive cells that are derived from peripheral blood, bone marrow, or the like. This cell population promotes angiogenesis and contributes to revascularization with the ability to eventually differentiate into endothelial cells (Non Patent Literature 1).

The number of endothelial progenitor cells in peripheral blood is reported to be inversely correlated with the degree of cardiovascular disease risk (Non Patent Literature 2) and to be inversely correlated with the mortality of coronary artery disease patients (Non Patent Literature 3). This suggests that the endothelial progenitor cells play an important role in angiogenesis in ischemic areas.

The CD34-positive cell content is approximately 100 times higher in bone marrow mononuclear cells than peripheral blood mononuclear cells. Clinically, revascularization (angiogenesis) therapy using autologous bone marrow cells has been practiced for patients with peripheral arterial disease (human ischemic limbs associated with arteriosclerosis obliterans (ASO) or Buerger's disease) and reportedly produced favorable outcomes (Non Patent Literature 4). In consideration of burdens on patients, however, it is more preferred to transplant endothelial progenitor cells derived from peripheral blood mononuclear cells rather than bone marrow cells. In this regard, another method has been attempted, which involves forcedly mobilizing endothelial progenitor cells from bone marrow to peripheral blood using VEGF, SDF, G-CSF, or the like.

Meanwhile, the present inventors have found that the transplantation of endothelial progenitor cells suppresses cancer cell growth, significantly altering the construction of tumor vessels, and reported anticancer therapy using endothelial progenitor cells (Patent Literature 1).

Regenerative medicine using autologous cells is advantageous in terms of ethics and safety. Unfortunately, diabetes mellitus or chronic renal failure patients or elderly people with a high risk of cardiovascular disease have the decreased numbers of endothelial progenitor cells with deteriorated functions (Non Patent Literatures 5 to 7). Thus, the cells cannot be prepared in an amount necessary for transplantation. In response to such problems, there have been reports on a method involving allowing endothelial progenitor cells to grow *in vitro* (Patent Literature 2) and a method involving inducing the differentiation of immature stem cells to endothelial progenitor cells by the action of Notch ligands (Patent Literature 3).

For effective regenerative medicine using endothelial progenitor cells few in number, it is desirable to improve the deteriorated functions of patient-derived endothelial progenitor cells and prepare highly functionalized endothelial progenitor cells. In one method, the functions of endothelial progenitor cells may be enhanced by stimulation with statin or prostacyclin. Disadvantageously, this method merely produces temporary effects that are not sustainable. Another possible method enhances the functions of endothelial progenitor cells by introduction of, for example, the VEGF or TERT gene. This method, however, improves only some of the functions of EPCs and fails to achieve the effect of improving the overall functions of EPCs. Thus, this method cannot bring about improvement leading to clinical application.

Apurinic/apyrimidinic endonuclease 1 (Ape1), also known as HAP1 or Ref-1, is an AP endonuclease that cleaves the phosphodiester backbone immediately 5' to the AP site through hydrolysis to produce a single-stranded DNA fragment having 3'-hydroxyl and 5'-deoxyribose phosphate ends.

Some reports suggest, as to the biological functions of Ape1, antitumor effects brought about by the inhibition of Ape1. For example, a low-molecular-weight Ape1 inhibitor (E3330) reportedly suppresses the growth and functions of HUVEC or EPC (Non Patent Literature 8). Introduction of a Tat-tagged Ape1 into endothelial cells reportedly suppresses the expression of the cell adhesion molecule VCAM-1 induced by TNF-α, suppressing the adhesion of mononuclear cells (Non Patent Literature 9).

All of these findings, however, suggest that Ape1 acts suppressively on endothelial progenitor cells.

### Citation List

### Patent Literature

Patent Literature 1: WO2008/142862
Patent Literature 2: Japanese Patent Laid-Open No. 2009-189743
Patent Literature 3: Japanese Patent Laid-Open No. 2007-89536 Non Patent Literature

Non Patent Literature 1: Science, 1997, 275, 964-967
Non Patent Literature 2: New Eng J Med 2003, 348, 593-600
Non Patent Literature 3: New Eng J Med 2005, 353, 999-1007
Non Patent Literature 4: Lancet 2002, 360, 427-435
Non Patent Literature 5: Circulation, 2002, 106, 2781-2786
Non Patent Literature 6: Arterio Thromb Vasc Biol, 2006, 2140-2146
Non Patent Literature 7: Diabetes, 2007, 56, 1559-1568
Non Patent Literature 8: Journal of Cellular Physiology. 2008, 209-218
Non Patent Literature 9: Biochemical and Biophysical Communications, 2008, 368, 68-73

### Summary of Invention

### Technical Problem

An object of the present invention is to improve the functions of stem cells/progenitor cells, particularly, endothelial progenitor cells or mesenchymal stem cells, thereby realizing more effective revascularization (angiogenesis) therapy using the limited number of patient-derived stem cells/progenitor cells.

### Solution to Problem

The present inventors have conducted various studies aimed at highly functionalizing endothelial progenitor cells (EPCs) and consequently found that the functions of endothelial progenitor cells are linked to their expression of the Ape1 gene. The present inventors have further confirmed that the functions of endothelial progenitor cells can be increased significantly by forcing the cells to express the Ape1 gene and that the administration of these highly functionalized endothelial progenitor cells to animal experimental models enhances revascularization effects.

The present inventors have also confirmed that introduction of the Ape1 gene into a mesenchymal stem cell line or myocardial stem cells relieves the oxidative stress-induced injury of the cells, as in the endothelial progenitor cells.

These results indicate that the stem cells/progenitor cells (e.g., endothelial progenitor cells, mesenchymal stem cells, or myocardial stem cells) highly functionalized by Ape1 are useful in improving the regenerative therapy of tissues or organs.

The present invention is defined as follows:
1. A cell preparation for use in a method of treatment selected from revascularization, organ regeneration, prevention or treatment of cancer, or prevention or treatment of ischemic diseases including lower limb ischemia, myocardial infarction, and cerebral infarction, the cell preparation comprising an endothelial progenitor cell or a mesenchymal stem cell wherein said method comprises a step of increasing Ape1 expression in said cells such that the cells have an improved ability to regenerate or generate an organ, or an improved revascularization or angiogenic capacity.
2. The cell preparation for use in a method according to item 1, wherein increased expression of Ape1 is based on induction of Ape1 expression.
3. The cell preparation for use in a method according to item 2, wherein the induction of Ape1 expression is based on introduction of an Ape1 gene.
4. The cell preparation for use in a method according to item 2, wherein the induction of Ape1 expression is based on introduction of an Ape1 protein.
5. The cell preparation for use in a method according to item 2, wherein the induction of Ape1 expression is based on an Ape1 expression inducer.
6. The cell preparation for use in a method according to item 5, wherein the Ape1 expression inducer is one or more selected from TNF-α, IL-1β, and IF-γ.
7. The cell preparation for use in a method according to any one of items 1 to 6, wherein the cell is derived from a patient in need of treatment using the cell preparation.
8. The cell preparation for use in a method according to any one of items 1 to 7, wherein the cell preparation is intravenously administered, intramuscularly administered, or applied directly to a tissue.
9. The cell preparation for use in a method according to any one of items 1 to 8, wherein the stem cell/progenitor cell is an endothelial progenitor cell.
10. The cell preparation for use in a method according to any one of items 1 to 9, wherein the stem cell/progenitor cell is a mesenchymal stem cell.
11. The cell preparation for use in a method according to any one of items 1 to 10, wherein the cell preparation is in a sheet form.
12. An *in vitro* method for improving the ability of an endothelial progenitor cell or mesenchymal stem cell to regenerate or generate an organ, comprising increasing expression of apurinic/apyrimidinic endonuclease 1 (Ape1) in the cell.
13. An *in vitro* method for improving the revascularization or angiogenic capacity of an endothelial progenitor cell or mesenchymal stem cell, comprising increasing expression of apurinic/apyrimidinic endonuclease 1 (Ape1) in the cell.

Specifically, the present invention relates to the use of a stem cell/progenitor cell having increased expression of apurinic/apyrimidinic endonuclease 1 (Ape1).

Embodiments using endothelial progenitor cells, mesenchymal stem cells, or myocardial stem cells as the stem cell/progenitor cell are described in Examples described later.

Expression of Ape1 can be increased, for example, by induction of Ape1 expression, introduction of the Ape1 gene, or introduction of the Ape1 protein.

The induction of Ape1 expression can be achieved, for example, by the application of an Ape1 expression inducer. Examples of the Ape1 expression inducer that may be used can include TNF-α, IL-1β, and IF-γ.

The stem cell/progenitor cell for use of the present invention is improved in its angiogenic capacity by increased expression of Ape1. The stem cell/progenitor cell for use of the present invention is also improved in its vascular injury repairing action (re-endothelialization action) and/or oxidative stress resistance.

The stem cell/progenitor cell for use of the present invention can be used preferably in various approaches of regenerative medicine including angiogenesis and vascular repair by virtue of such high functionalization of the stem cell/progenitor cell.

The present invention also provides the use of a cell preparation comprising the stem cell/progenitor cell for use of the present invention.

For the cell preparation for use of the present invention, it is preferred that the stem cell/progenitor cell used should be derived from a patient in need of treatment.

The cell preparation for use of the present invention is used in revascularization, organ regeneration, prevention or treatment of cancer, or prevention or treatment of ischemic diseases including lower limb ischemia, myocardial infarction, and cerebral infarction.

The method for applying the cell preparation for use of the present invention is not particularly limited. For example, the cell preparation for use of the present invention is administered directly to an affected area by intravenous administration or intramuscular administration. Alternatively, the cell preparation for use of the present invention is processed, for example, into a sheet form and used by direct application to a tissue.

The form of the cell preparation for use of the present invention is not particularly limited. As described above, the cell preparation for use of the present invention may be processed into a sheet form that can be applied directly to a tissue.

The present disclosure also provides revascularization therapy comprising administering the cell preparation for use of the present invention to a patient.

The present disclosure further provides a method comprising functionally evaluating a stem cell/progenitor cell with an apurinic/apyrimidinic endonuclease 1 (Ape1) or Ape1 gene expression level in the stem cell/progenitor cell as an indicator, and a kit therefor (kit for evaluating function of a stem cell/progenitor cell).

The kit comprises, for example, at least one of the following components (a) to (c):
(a) an anti-Ape1 antibody,
(b) oligonucleotide primers for specifically binding to the Ape1 gene and amplifying the gene, and
(c) a polynucleotide probe for specifically binding to the Ape1 gene and detecting the gene.

### Advantageous Effects of Invention

The present invention can enhance the abilities of stem cells/progenitor cells to regenerate or generate tissues or organs, such as a vascular injury repairing action (re-endothelialization), an angiogenic action, and oxidative stress resistance, and can improve the effects of regenerative therapy using the cells. The present disclosure can also achieve convenient functional evaluation of stem cells/progenitor cells with Ape1 expression as an indicator. The evaluation results can be applied to preoperative evaluation for cell-based revascularization therapy or the diagnosis of angiopathic disease.

### Brief Description of Drawings

[Figure 1] Figure 1 shows the abilities of diabetic mouse (DM)- and aged mouse (Aged)-derived endothelial progenitor cells to adhere.
[Figure 2] Figure 2 shows results of comparing the expression level of the Ape1 gene between the endothelial progenitor cells of an aged mouse (1 year and 6 months old: Aged-EPC) and young mice (12-14 weeks old: Normal-EPC).
[Figure 3] Figure 3 shows the expression of Ape1 in normal endothelial progenitor cells that are not transfected (Figure 3A: Non-transfected EPCs) and endothelial progenitor cells that are transfected with the Ape1 gene (Figure 3B: Standard Method, Figure 3C: Magnet Transfection Method).
[Figure 4] Figure 4 shows the vascular remodeling effect of Ape1-introduced endothelial progenitor cells after femoral arterial injury in a mouse. Figure 4 (A) shows HE-stained images of an untreated sample (None), a sample that received confluent EPCs (ct EPC), and a sample that received Ape1 gene-introduced EPCs (Ape-EPC) in this order from the left. Figure 4(B) shows the I/M ratios of an untreated sample (None), a sample that received confluent EPCs (ct EPC), and a sample that received Ape1 gene-introduced EPCs (Ape-EPC) in this order from the left.
[Figure 5] Figure 5 shows that introduction of Ape1 relieves pericyte-derived mesenchymal stem cells from the oxidative stress-induced injury (○ : cells infected with human Ape1, ●: control cells infected with LacZ-expressing adenovirus).
[Figure 6] Figure 6 shows results of measuring the amounts of ROS and superoxide in human-derived myocardial stem cells by flow cytometry.

### Description of Embodiments

The present invention relates to the use of a stem cell/progenitor cell superior in functions (e.g., angiogenic capacity) to normal stem cells/progenitor cells by virtue of increased expression of Ape1. Hereinafter, the stem cell/progenitor cell for use of the present invention is referred to as a "highly functionalized stem cell/progenitor cell". Particularly, the endothelial progenitor cell for use of the present invention is referred to as a "highly functionalized endothelial progenitor cell". Also, the mesenchymal stem cell for use of the present invention is referred to as a "highly functionalized mesenchymal stem cells".

### 1. Stem cell/progenitor cell

In the present invention, the "stem cell" refers to a cell having both of the ability to differentiate into multiple lineages of cells (multipotency or pluripotency) and the ability to maintain its multipotency or pluripotency even after cell division (ability to self-renew) and encompasses various stem cells that reside in each tissue *in vivo*, for example, hematopoietic stem cells, neural stem cells, hepatic stem cells, dermal stem cells, germ stem cells, and ES or iPS cells, and stem cells induced from these cells. The "progenitor cell" refers to a cell in an intermediate stage of the process in which the stem cell differentiates into a specific somatic cell or germ cell, and encompasses various progenitor cells, as in the stem cell. In the present invention, the stem cell/progenitor cell is selected from endothelial progenitor cells and mesenchymal stem cells described later.

### 1.1 Endothelial progenitor cell

The "endothelial progenitor cells (EPCs)" refer to a population of cells that are derived from bone marrow, peripheral blood, umbilical cord blood, or the like. This cell population promotes angiogenesis and contributes to revascularization with the ability to eventually differentiate into endothelial cells.

At the moment, the endothelial progenitor cells cannot be more clearly defined. More strictly, the endothelial progenitor cells are a heterogenous cell population in which cells having the ability to differentiate into endothelial cells coexist with cells that promote the construction or formation of new blood vessels via the production of various cytokines or the like without directly differentiating into endothelial cells.

The endothelial progenitor cells can be separated and collected from bone marrow as well as peripheral blood, umbilical cord blood, or the like.

The endothelial progenitor cells in peripheral blood are usually identified as a CD34/KDR-positive cell population in the case of humans and as cKit/Flk-positive cells (mice) in the case of mice.

CD34+ cells (in the case of humans) or Lin-/cKit+ or Sca1+ cells (in the case of mice) mean every "undifferentiated cell" including so-called hematopoietic stem cells. Among these undifferentiated mononuclear cells, cells that adhere to a fibronectin-coated culture dish by culture in a VEGF-containing medium on the culture dish and further form a colony after growth (fibronectin-adhesive cells having the ability to adsorb acetyl LDL and the ability to bind to lectin) is considered as a cell population rich in the endothelial progenitor cells.

The endothelial progenitor cells may be separated from mononuclear cells by means of magnetic beads, flow cytometry, or the like using the surface markers described above. As described above, markers for accurately identifying the endothelial progenitor cells have not yet been found, and the separation using two or three markers have certain limitations. The present inventors have isolated mouse endothelial progenitor cells on the basis of Lin negative, cKit positive, and Flk positive.

Alternatively, the endothelial progenitor cells can be prepared by a method involving culturing mononuclear cells separated from peripheral blood or bone marrow using an endothelial differentiation promotion medium containing a cytokine such as VEGF, thereby collecting endothelial progenitor cells as adherent cells.

Also, there have been reported a method involving coculturing undifferentiated bone marrow cells and cells highly expressing Notch ligands such as Jagged-1 or Delta-4 in an adjacent state to induce the differentiation of the undifferentiated bone marrow cells into endothelial progenitor cell-like cells, and obtaining the differentiated cells (Japanese Patent Laid-Open No. 2007-89536) and a method involving collecting endothelial progenitor cells using a cell separation filter (Japanese Patent Laid-Open No. 2003-250820). The endothelial progenitor cells may be prepared using such a method known in the art.

Recently, revascularization therapy, which involves transplanting bone marrow-derived endothelial progenitor cells, has been practiced for patients with ischemic disease or arterial occlusive disease and produced favorable outcomes. The endothelial progenitor cells used in this treatment are CD34-positive mononuclear cells derived from bone marrow.

In addition to the cells prepared by the methods described above, endothelial progenitor cells inductively differentiated from induced pluripotent stem cells typified by iPS cells can also be used preferably as a source of the highly functionalized endothelial progenitor cell according to the present invention.

The endothelial progenitor cells are known to have functions such as an angiogenic effect, a vascular injury repairing action (re-endothelialization action), and oxidative stress resistance and as such, are used in cell-based revascularization therapy for ischemic disease or arterial occlusive disease.

The present inventors have also found that the transplantation of endothelial progenitor cells suppresses cancer cell growth, significantly altering the construction of tumor vessels, and reported anticancer therapy using endothelial progenitor cells (WO2008/142862). Specifically, the endothelial progenitor cells play an important role in the cell transplantation therapy of various diseases associated with abnormal angiogenesis.

Unfortunately, only the limited number of endothelial progenitor cells can be obtained from each patient. Endothelial progenitor cells contained in peripheral blood, which can be collected without particular burdens on the patient, are few in number. Such patient-derived endothelial progenitor cells tend to have deteriorated functions and thus, are insufficiently effective even if transplanted.

If the limited functions of endothelial progenitor cells can be increased, this contributes to improvement in regenerative medicine using the endothelial progenitor cells.

### 1.2 Mesenchymal stem cells

The mesenchymal stem cells refer to somatic stem cells derived from mesenchyme. These cells have the ability to differentiate into cells belonging to the mesenchymal system and as such, can be expected to be applied to the regenerative medicine of bones, blood vessels, or cardiac muscles.

The mesenchymal stem cells are thought to reside in every tissue containing mesenchymal tissues. Bone marrow mesenchymal stem cells are contained in bone marrow stromal cells. The bone marrow stromal cells are one type of cells supporting hematopoietic cells that are principal constituents in bone marrow. The bone marrow stromal cells assume a network structure for supporting hematopoietic cells in bone marrow. The bone marrow is a principal hematopoietic organ that produces blood cells *in vivo.*

The mesenchymal stem cells are also found in pericytes (also called perithelial cells) that reside to surround capillary walls. The pericytes are cells of mesodermal origin also called Rouget cells. In Examples described later, the effects of forcedly expressed Ape1 were verified using a mesenchymal stem cell line prepared from this pericyte.

### 2. Apurinic/apyrimidinic endonuclease 1 (Ape1)

The apurinic/apyrimidinic endonuclease 1 (Ape1), also known as APEX1 (official gene symbol), HAP1, or Ref-1, is an AP endonuclease that cleaves the phosphodiester backbone immediately 5' to the AP site through hydrolysis to produce a single-stranded DNA fragment having 3'-hydroxyl and 5'-deoxyribose phosphate ends.

Ape1 has also been reported to have such AP endonuclease activity as well as weak DNA 3'-diesterase activity, 3'->5' exonuclease activity, and RNase H activity. Ape1 has not only DNA repair activity but also the *in vitro* function of adjusting the DNA binding activities of many transcriptional factors through the redox mechanism. In this regard, Ape1 is a molecule responsible for the function of protecting a cell against oxidative stress to the cell.

The nucleotide sequence of the Ape1 gene and the amino acid sequence of Ape1 have already been known in the art and published in a public database such as GenBank. For example, the nucleotide sequence (mRNA) and amino acid sequence of human Ape1 are publicly available under Accession Numbers NM_001641.2 and NP_001632.2 (variant 1), NM_080648.1 and NP_542379.1 (variant 2), or NM_080649.1 and NP_542380.1 (variant 3) for each of three variants. Also, the nucleotide sequence and amino acid sequence of mouse Ape1 are publicly available under Accession Numbers NM_009687.1 and NP_033817.1.

The Ape1 gene can be obtained by extracting RNAs from cells and amplifying the gene of interest using primers designed on the basis of the sequence described above. One example of the primers is as follows:
Forward Primer: 5-GGA TTG GGT AAA GGA AGA AGC A-3 (commonly for mice and rats; SEQ ID NO: 1)
   5-GTG CCC ACT CAA AGT TTC TTA C-3 (for humans; SEQ ID NO: 2)
Reverse Primer: 5-CAA GGC GCC AAC CAA CAT TCT T-3 (commonly for humans, mice, and rats; SEQ ID NO: 3)

In search for genes involved in the functions of endothelial progenitor cells, the present inventors have found that Ape1 expressed in endothelial progenitor cells plays an important role in the maintenance of the cells against oxidative stress and in the angiogenic capacity of the cells. The present inventors have further confirmed that introduction of Ape1 into a mesenchymal stem cell line and myocardial stem cells may relieve the injury of the cells.

Specifically, the high expression of Ape1 enhances resistance to oxidative stress and brings about the effects of promoting re-endothelialization and promoting angiogenesis in injured vascular walls. Hence, increasing expression of Ape1 can increase the viability of stem cells/progenitor cells in an oxidative stress environment and allow the cells to sufficiently exert their "re-endothelialization" and "angiogenic" actions in local areas such as ischemic areas or injured blood vessels.

### 3. Highly functionalized stem cell/progenitor cell

### 3.1 Increased expression of Ape1

The stem cell/progenitor cell for use according to the present invention is a highly functionalized stem cell/progenitor cell having increased expression of Ape1. In this context, the phrase "having increased expression of Ape1" encompasses both of direct increase in Ape1 activity attributed to the increased expression level of the Ape1 protein and indirect increase in Ape1 activity attributed to, for example, the cancellation of a related inhibitory system.

For example, increased expression of Ape1 can be brought about by induction of Ape1 expression (induction of expression of the Ape1 gene or the Ape1 protein) using an external factor. Examples of the method for inducing Ape1 expression include introduction of the Ape1 gene, introduction of the Ape1 protein, and methods using an Ape1 expression inducer. Alternatively, the stem cell/progenitor cell having increased expression of Ape1 may be obtained without use of the external factor by particularly selecting cells having high Ape1 activity from a heterogenous stem cell/progenitor cell population and isolating the selected cells, followed by amplification.

In the former case, the phrase "*having increased expression of* Ape1" means the high expression of Ape1 in the cells after the induction of Ape1 expression, compared with untreated stem cells/progenitor cells. In the latter case, the phrase "having increased expression of Ape1" means the higher expression of Ape1 in the selected and amplified cells than the average Ape1 expression of the stem cell/progenitor cell population from which those cells are derived.

The expression level of the Ape1 protein is not particularly limited and is desirably improved by 5 or more times, preferably 10 or more times, compared with reference cells.

In Examples described later, all endothelial progenitor cells, mesenchymal stem cells, or myocardial stem cells were infected with Ape-adenovirus. The Ape expression level was confirmed to rise by about 2 to 5 times in all of these cells, compared with control cells. Since the rate of infection was presumably on the order of 20%, the estimated Ape1 expression level rose by about 10 to 20 times in the infected cells.

### 3.2 High functionalization of stem cell/progenitor cell

In the present invention, the term "highly functionalized" or "high functionalization" means that the resulting stem cell/progenitor cell is improved in its functions (e.g., the ability to regenerate or generate an organ (e.g., revascularization or angiogenic capacity) and oxidative stress resistance) or viability. In the present invention, the high functionalization of the stem cell/progenitor cell is brought about by increased expression of Ape1, as described above.

In the case of using, for example, patient-derived cells, the cells are more improved in their angiogenic capacity, oxidative stress resistance, and the like by increased expression of Ape1 than cells with deteriorated functions of the patient and are thus therapeutically useful (highly functionalized). Preferably, the cells used are highly functionalized to a level equal to or higher than that of stem cells/progenitor cells derived from healthy persons or normal controls (young persons in their thirties, a disease-free cohort, etc.).

Hence, the cells obtained even in trace amounts from the patient can achieve a high revascularization (angiogenic) effect.

### 4. Preparation of highly functionalized stem cell/progenitor cell

The highly functionalized stem cell/progenitor cells for use of the present invention can be prepared, for example, by induction of Ape1 expression.

### 4.1 Introduction of Ape1 gene

The induction of Ape1 expression can be performed by induction of the Ape1 gene into stem cells/progenitor cells.

The Ape1 gene can be prepared on the basis of the sequence known in the art according to a routine method. For example, RNAs are extracted from bone marrow- or peripheral blood-derived cells. Primers can be prepared on the basis of the sequence known in the art and used in cloning by PCR to prepare the cDNAs of the Ape1 gene of interest.

In the present invention, the Ape1 gene can be introduced into the cells using a method usually used in the transfection of animal cells, for example, calcium phosphate method, lipofection, electroporation, microinjection, or a method using retrovirus or baculovirus as a vector. A method using adenovirus, adeno-associated virus, or retrovirus vectors is preferred in terms of safety and efficiency of introduction.

The virus vectors can be prepared on the basis of the method of Miyake et al. (Miyake, S. et al., Proc. Natl. Acad. Sci. 93: 1320-1324 (1993)). Alternatively, commercially available Adenovirus Cre/loxP Kit (manufactured by Takara Shuzo Co., Ltd.) may be used. This kit is a kit for preparing recombinant adenovirus vectors based on a novel expression control system using the Cre recombinase of P1 phage and its recognition sequence loxP (Kanegae Y. et.al., 1995 Nucl. Acids Res. 23, 3816) and can conveniently prepare Ape1 gene-incorporated recombinant adenovirus vectors.

The multiplicity of infection (MOI) of adenovirus infection depends on a gene insert and cells for the introduction and must thus be determined appropriately. To introduce the gene into stem cells/progenitor cells, the Ape1 gene-recombinant adenovirus preferably has MOI of 10 to 500 (more preferably around 100).

### 4.2 Introduction of Ape1 protein

The induction of Ape1 expression may be performed by introduction of the Ape1 protein into stem cells/progenitor cells.

Introduction of the Ape1 protein is performed according to a method known in the art. Specifically, Ape1 is introduced into stem cells/progenitor cells together with a protein introduction reagent. Alternatively, a protein transfection domain known in the art (see e.g., Japanese Patent Laid-Open Nos. 2005-287418 and 2005-110565) is fused to the N terminus of the Ape1 protein, which is then introduced into the cells. In this context, the "protein transfection domain" is not particularly limited as long as the domain is a peptide or the like capable of assisting protein introduction. Examples of the protein transfection domain can include HIV TAT (Green and Loewenstein, Cell, 56 (6): 1179-88 (1988); and Frankel and Pabo, Cell, 55 (6): 1189-93 (1988)), antennapedia homeodomain (Vives et al., J. Biol. Chem, 272 (25) : 16010-7 (1997)), HSV VP22 (Elliott and O'Hare, Cell, 88 (2) : 223-33 (1997)), and cell penetrating peptides (CPPs) or fragments thereof. Alternatively, substances binding to receptors specifically expressed in stem cells/progenitor cells for the introduction may be used as the "protein transfection domain".

### 4.3 Ape1 expression inducer

The induction of Ape1 expression can also be performed by the introduction of an "Ape1 expression inducer" capable of exogenously inducing Ape1 expression in the stem cells/progenitor cells of interest.

Examples of the "Ape1 expression inducer" can include TNF-α, IL-1β, and IFN-γ. These "Ape1 expression inducers" are added to a medium for stem cell/progenitor cell culture described later, and stem cells/progenitor cells are cultured therein by a usual method. The culture time is at least 6 hours, preferably 24 to 48 hours, though the culture time is not limited thereto. The amount of the Ape1 expression inducer added to the medium is appropriately set and is, for example, 1 to 100 ng/ml, preferably approximately 5 ng/ml, of TNF-α.

### 5. Method for culturing highly functionalized stem cell/progenitor cell

The highly functionalized stem cell/progenitor cell for use of the present invention can be cultured for growth by a method similar to the culture method of normal stem cells/progenitor cells.

The medium is not particularly limited as long as the medium is suitable for the culture of mononuclear cells. A MEM, BME, DME, α-MEM, IMEM, ES, DM-160, Fisher, F12, WE, RPMI, StemSpan, or StemPro medium or a mixture thereof can be used as a basal medium. Alternatively, a commercially available medium for lymphocyte culture (e.g., GT-T medium (Takara Bio Inc.), AIM V medium (Invitrogen Corp.), medium for T-lymphocyte culture (Cosmo Bio Co., Ltd.), X-VIVO medium (manufactured by Lonza Group AG), ECM medium, and ECM-MV2 medium) or a commercially available medium for endothelial cells (e.g., EGM-2 medium or EBM-2 medium) may be used. The basal medium needs to be supplemented with VEGF and heparin.

The medium may be further supplemented appropriately with various nutrients necessary for cell maintenance and growth and each component necessary for induction of differentiation. The medium can contain, for example, nutrients including: carbon sources such as glycerol, glucose, fructose, sucrose, lactose, honey, starch, and dextrin; hydrocarbons such as fatty acid, fat and oil, lecithin, and alcohols; nitrogen sources such as ammonium sulfate, ammonium nitrate, ammonium chloride, urea, and sodium nitrate; inorganic salts such as sodium salt, potassium salt, phosphate, magnesium salt, calcium salt, iron salt, and manganese salt; monopotassium phosphate, dipotassium phosphate, magnesium sulfate, sodium chloride, ferrous sulfate, sodium molybdate, sodium tungstate, and manganese sulfate; various vitamins; and amino acids.

The medium is preferably a "serum-free medium", which does not contain animal serum such as FBS or FCS. The medium may be supplemented with a serum substitute, knockout serum replacement (KSR), or the like.

The medium obtained by mixing these components has pH in the range of 5.5 to 9.0, preferably 6.0 to 8.0, more preferably 6.5 to 7.5.

The endothelial progenitor cells or the mesenchymal stem cells are adherent cells having the property of adhering to an extracellular matrix for their growth. The highly functionalized endothelial progenitor cell for use of the present invention also has similar properties. Hence, it is preferred for the cell culture to use an appropriate scaffold. The scaffold is not particularly limited as long as the scaffold is a matrix, a substrate, or a carrier to which cells can adhere for their division and growth. Examples of the scaffold can include fibronectin, vitronectin, collagen, proteoglycan, laminin, tenascin, entactin, elastin, fibrillin, hyaluronic acid, gelatin, poly-L-lysine, and poly-D-lysine. Particularly, it is desirable to use fibronectin as a matrix.

The cells are cultured at 36°C to 38°C, preferably 36.5°C to 37.5°C, under conditions of 1% to 25% O₂ and 1% to 15% CO₂ with the medium replaced by an appropriate one.

### 6. Cell preparation comprising highly functionalized stem cell/progenitor cell for use

The highly functionalized stem cell/progenitor cell for use of the present invention is excellent in angiogenic effect, vascular injury repairing action (re-endothelialization action), and oxidative stress resistance and can be used preferably in revascularization medicine for ischemic disease, arterial occlusive disease, or the like as defined in claim 1. Specifically, the present invention provides the use of a cell preparation comprising the highly functionalized stem cell/progenitor cell.

The method for administering the cell preparation for use of the present invention is not particularly limited. A possible administration method is, for example, local transplantation by surgical means, intravenous administration, administration through lumbar puncture, local infusion, hypodermic administration, intradermal administration, intraperitoneal administration, intramuscular administration, intracerebral administration, intracerebroventricular administration, or venous administration, according to an application site.

Endothelial progenitor cells in blood accumulate to a vascular injury site by their own properties. This means that the endothelial progenitor cells have a property of specifically accumulating to an ischemic area and promoting the revascularization of the injured area even when intravenously administered to a site distant from the affected area. Hence, the cell preparation for use of the present invention can achieve, even by convenient venous administration, the revascularization therapy of an ischemic area distant from the administration site without placing burdens on the patient. In order to excert an angiogenic action on a peripheral organ with ischemia, the endothelial progenitor cells may be intramuscularly administered to the local area in the case of blood flow insufficient for the delivery of the cells.

The preparation for use of the present invention may be in a sheet form and applied directly to an affected area. The sheet may comprise not only the cell but also an appropriate support.

The cell preparation for use of the present invention may further comprise scaffold materials or components assisting maintenance, growth and administration to an affected area of the cells, or other pharmaceutically acceptable carriers. Examples of the components necessary for cell maintenance and growth include: medium components such as carbon sources, nitrogen sources, vitamins, minerals, salts, and various cytokines; and extracellular matrix formulations such as Matrigel(TM).

Examples of the scaffold materials or components assisting administration to an affected area include: biodegradable polymers such as collagen, polylactic acid, hyaluronic acid, cellulose, and derivatives thereof, and complexes consisting of two or more thereof; and injectable aqueous solutions such as saline, media, physiological buffer solutions such as PBS, and isotonic solutions (e.g., D-sorbitol, D-mannose, D-mannitol, and sodium chloride solutions) containing glucose and other aids. These materials or components may be used in combination with an appropriate solubilizer, for example, an alcohol (specifically, ethanol), a polyalcohol (e.g., propylene glycol or polyethylene glycol), or a nonionic surfactant (e.g., polysorbate 80 or HCO-50).

The cell preparation for use of the present invention may further comprise a pharmaceutically acceptable organic solvent, polyvinyl alcohol, polyvinylpyrrolidone, a carboxyvinyl polymer, sodium carboxymethylcellulose, sodium polyacrylate, sodium alginate, water-soluble dextran, sodium carboxymethyl starch, pectin, methylcellulose, ethylcellulose, xanthan gum, gum arabic, casein, agar, polyethylene glycol, diglycerin, glycerin, propylene glycol, Vaseline, paraffin, stearyl alcohol, stearic acid, mannitol, sorbitol, lactose, and acceptable pharmaceutical additives such as surfactants, buffers, emulsifiers, suspending agents, soothing agents, and stabilizers, if necessary.

Actual additives are selected singly or in appropriate combination from among those described above according to the dosage form of the therapeutic agent for use of the present invention, though the additive are not limited thereto. In the case of using, for example, a preparation for injection, purified antibodies are suspended in a solvent, for example, saline, a buffer solution, or a glucose solution, which can then be supplemented with an adsorption-preventing agent, for example, Tween 80, Tween 20, or gelatin and used.

Examples of the disease targeted by the cell preparation for use of the present invention include: ischemic diseases such as myocardial infarction and cerebral infarction; and peripheral arterial occlusive disease.

The present inventors have found that the transplantation of endothelial progenitor cells suppresses cancer cell growth, significantly altering the construction of tumor vessels, and reported anticancer therapy using endothelial progenitor cells (supra). Hence, the cell preparation for use of the present invention can also be used preferably in such prevention or treatment of cancer.

The cell preparation for use of the present invention can be used in a method according to claim 1.

### 7. Functional evaluation of stem cell/progenitor cell

Conventional methods for evaluating function of endothelial progenitor cells involve, for example, examining the cells for their abilities to grow or migrate in a culture system or examining the cells *in vivo* for their abilities to repair vascular injury (re-endothelialization) or degrees of improvement in the blood flows of peripheral tissues (angiogenesis). These methods, however, are very complicated. Thus, a more simple method has been attempted, which involves measuring the expression level of a gene population closely related to the functions of endothelial progenitor cells to "predict" the functions of endothelial progenitor cells.

The present disclosure provides a method for conveniently and accurately functionally evaluating stem cells/progenitor cells (endothelial progenitor cells, mesenchymal stem cells, myocardial stem cells, etc.) by using the Ape1 gene as an analyte. Specifically, the expression level of the Ape1 gene or the Ape1 protein in stem cells/progenitor cells is measured and compared with the average expression level of the Ape1 gene or the Ape1 protein in cells of the same type thereas to functionally evaluate the stem cells/progenitor cells.

### 7.1 Measurement of Ape1 gene expression level

The expression level of the Ape1 gene is measured by first extracting total RNA from collected cells and measuring the expression level of the Ape1 gene (mRNA) in the total RNA by any method described later.

The method for extracting total RNA is not particularly limited. For example, guanidine thiocyanate-cesium chloride ultracentrifugation method, guanidine thiocyanate-hot phenol method, guanidine hydrochloride method, or acidic guanidine thiocyanate-phenol-chloroform method (Chomczynski, P. and Sacchi, N., (1987) Anal. Biochem., 162, 156-159) can be adopted. The extracted total RNA may be further purified into mRNA alone, if necessary.

The gene expression level can be measured using a method known in the art such as nucleic acid hybridization method using samples immobilized on solid phases such as gene chips or arrays, RT-PCR, real-time PCR, subtraction, differential display, differential hybridization, or cross hybridization.

### 7.2 Measurement of Ape1 protein expression level

The expression level of the Ape1 protein can be measured using, for example, an immunological method based on antigen-antibody reaction.

Examples of the immunological method can include: solid-phase immunoassay such as immunoprecipitation, Western blotting, dot blotting, slot blotting, ELISA, and RIA; modifications known in the art altered from these methods (sandwich ELISA, methods described in US Patent No. 4202875, the method of Meager et al. (Meager A., Clin Exp Immunol. 2003 Apr, 132 (1), p. 128-36), etc.). Specifically, on the basis of these methods, the Ape1 protein expression level is measured using an antibody specifically binding to the Ape1 protein.

The anti-Ape1 antibody may be labeled, if necessary. For this labeling, the anti-Ape1 antibody may be labeled directly or may be used as a primary antibody in cooperation with a labeled secondary antibody specifically recognizing the primary antibody (recognizing an antibody derived from an animal serving as an origin of the primary antibody). The type of the label is preferably an enzyme (alkaline phosphatase or horseradish peroxidase) or biotin (which however requires the additional procedure of binding enzyme-labeled streptavidin to biotin on the secondary antibody), though the label is not limited thereto. The labeled secondary antibody (or labeled streptavidin) is commercially available as various prelabeled antibody (or streptavidin) products. In the case of RIA, an antibody labeled with a radioisotope such as ¹²⁵I is used, and the expression level is measured using a liquid scintillation counter or the like.

The activity of the enzyme used as this label can be detected to measure the expression level of the antigen. In the case of labeling with alkaline phosphatase or horseradish peroxidase, substrates that develop color or light by the catalysis of these enzymes are commercially available.

In the case of using the color-developing substrate, the color can be detected by visual observation using Western blotting or dot/slot blotting. For ELISA, it is preferred to measure the absorbance (measurement wavelength differs depending on the substrate) of each well using a commercially available microplate reader to quantify the antigen. Alternatively, a dilution series of the antigen used in the antibody preparation is prepared and subjected as standard antigen samples to detection operation simultaneously with other samples. A calibration curve on which measured values are plotted against standard antigen concentrations may be prepared to quantify the antigen concentrations in the other samples.

In the case of using the light-developing substrate, the light can be detected in Western blotting or dot/slot blotting by autoradiography using X-ray films or imaging plates or by photography using an instant camera. Alternatively, the antigen may be quantified using densitometry, Molecular Imager Fx System (manufactured by Bio-Rad Laboratories, Inc.), or the like. In the case of using the light-developing substrate in ELISA, the enzymatic activity is measured using a luminescence microplate reader (manufactured by, e.g., Bio-Rad Laboratories, Inc.).

In the present invention, the "expression level" of the Ape1 gene or the KIAA protein serving as an indicator is not limited to the physical amount thereof and also includes activity and titer (antibody titer, etc.) indirectly exhibiting this expression level.

### 7.3 Evaluation

The stem cell/progenitor cell for use of the present invention can be functionally evaluated, for example, by using the average expression level of the Ape1 gene or the Ape1 protein in normal stem cells/progenitor cells as a reference value and comparing the reference value with the Ape1 gene or Ape1 protein expression level of the test cell. The Ape1 gene or Ape1 protein expression level may be measured after preliminary cell stimulation with the Ape1 expression inducer described above. In this case, the stem cell/progenitor cell can be functionally evaluated on the basis of the "ability to induce Ape1 expression".

When the Ape1 gene or Ape1 protein expression level of the test cell is lower than the reference value, this test cell can be evaluated as being a stem cell/progenitor cell having deteriorated functions.

Such results of functionally evaluating stem cells/progenitor cells can be used in, for example, preoperative evaluation for regenerative medicine using stem cells/progenitor cells such as endothelial progenitor cells, mesenchymal stem cells, or myocardial stem cells and can also be used in the evaluation of risk of developing various diseases associated with the deteriorated functions of these cells, for example, myocardial infarction, or the severity of these diseases.

### 8. Kit for evaluating function of stem cell/progenitor cell

The present disclosure also provides a reagent or kit for evaluating function of the stem cell/progenitor cell. The kit comprises, as an essential component, at least one of the following components (a) to (c):
(a) an anti-Ape1 antibody,
(b) oligonucleotide primers for specifically binding to the Ape1 gene and amplifying the gene, and
(c) a polynucleotide probe for specifically binding to the Ape1 gene and detecting the gene.

These components may be used alone as respective reagents for evaluating function of stem cells/progenitor cells.

### 8.1 Anti-Ape1 antibody

The anti-Ape1 antibody can be prepared according to a method known in the art. Specifically, an animal is immunized with the Ape1 protein as an antigen or its arbitrary partial polypeptide by a routine method. Antibodies produced *in vivo* by the animal can be collected and purified to obtain the anti-Ape1 antibody.

The antibody may be a polyclonal antibody and is preferably a monoclonal antibody. The monoclonal antibody can be obtained from hybridomas established according to a method known in the art (e.g., Kohler and Milstein, Nature 256, 495-497, 1975; and Kennett, R. ed., Monoclonal Antibodies p. 365-367, 1980, Plenum Press, N.Y.) by fusing antibody-producing cells that produce specific antibodies with myeloma cells.

Examples of the antigen for antibody preparation can include the Ape1 protein as an antigen or its partial polypeptide (epitope peptide) consisting of a partial sequence with at least 6 consecutive amino acids of the Ape1 protein, and derivatives having an arbitrary amino acid sequence or carrier (e.g., keyhole limpet hemocyanin attached to the N terminus) attached to the Ape1 protein or the partial polypeptide.

The antigenic polypeptide can be obtained by allowing host cells to produce the Ape1 protein or its partial polypeptide (epitope peptide) by genetic engineering. Specifically, a vector capable of expressing the Ape1 gene or a portion thereof can be prepared and introduced into host cells to express the gene.

The anti-Ape1 antibody may be labeled appropriately (e.g., enzymatically, radioactively, or fluorescently) or may be modified appropriately with biotin or the like. The anti-Ape1 antibody may also be immobilized on an appropriate solid-phase support. Alternatively, such a support that permits solid-phase immobilization may be contained additionally in the kit. Examples of such a support that may be used include: synthetic resins to which proteins can adhere, such as polyethylene, polypropylene, polybutylene, polystyrene, polymethacrylate, and polyacrylamide; supports made of glass, nitrocellulose, cellulose, and agarose; and gel-type supports. The form of the support is not particularly limited, and the support is provided in the form of microparticles such as microspheres or beads (e.g., "latex" beads), a tube (inner wall) such as a microcentrifuge tube, a microtiter plate (well), or the like.

### 8.2 Primers for Ape1 gene amplification

The primers for Ape1 gene amplification are oligonucleotides of 5 to 30 consecutive bases in length having a sequence complementary to at least a portion of the Ape1 gene. The primers can be designed easily according to a routine method, for example, using commercially available primer design software, on the basis of the nucleotide sequence for Ape1 gene amplification (SEQ ID NO: 1) and prepared by amplification. Examples of such primers can include oligonucleotides having nucleotide sequences represented by SEQ ID NOs: 1 to 5, respectively.

The primers for Ape1 gene amplification may be labeled appropriately (e.g., enzymatically, radioactively, or fluorescently) or may be modified with biotin, phosphoric acid, amine, or the like.

### 8.3 Probe for Ape1 gene detection

The probe for Ape1 gene detection is a polynucleotide specifically hybridizing to the Ape1 gene and is preferably approximately 20 to 1500 bases long. Specifically, a single-stranded oligonucleotide or double-stranded DNA of approximately 20 bases in length is preferably used in Northern hybridization. Alternatively, a double-stranded DNA of approximately 100 to 1500 bases in length or a single-stranded oligonucleotide of approximately 20 to 100 bases in length is preferably used in a microarray. In the case of Affymetrix Gene Chip system, a single-stranded oligonucleotide of approximately 25 bases in length is preferred. For these probes, it is preferred to design probes specifically hybridizing to a site with high sequence specificity particularly located in the 3' noncoding region of the Ape1 gene.

The probe for Ape1 gene detection may be labeled appropriately (e.g., enzymatically, radioactively, or fluorescently) or may be modified with biotin, phosphoric acid, amine, or the like.

The primers for Ape1 gene amplification and the probe for Ape1 gene detection may be labeled appropriately (e.g., enzymatically, radioactively, or fluorescently) or may be modified with biotin, phosphoric acid, amine, or the like. The probe for Ape1 gene detection may also be immobilized on an appropriate support such as a glass plate, a nylon membrane, microbeads, or a silicon chip.

The kit of the present disclosure may further comprise, in addition to the components described above, other factors necessary for Ape1 detection, such as a (labeled) secondary antibody specific for the anti-Ape1 antibody, a reagent for detection of the labeling material, a buffer solution for reaction, an enzyme, and a substrate.

### Examples

Hereinafter, the present invention will be described specifically with reference to Examples. However, the present invention is not intended to be limited to these Examples.

### Example 1: Endothelial progenitor cell (EPC)

### 1. Deteriorated function of endothelial progenitor cell (EPC) in diabetic and aged mice

Mononuclear cells were isolated from the bone marrows of a diabetic (DM) mouse and an aged (Aged: 1 year and 6 months old) mouse. Then, Lin-, cKit+, and Flk+ cells were purified by a magnet sorting system (MACS). The purified EPCs were cultured for 48 hours in a fibronectin-coated α-MEM (GIBCO) (or Endothelial cell basal medium EBM (Clontech)) medium containing 10% FCS and VEGF. Of the cells that adhered thereto, acetyl-LDL adsorption- and lectin binding-positive cells were selected as adhesive EPCs and evaluated for their abilities to adhere to fibronectin, which are one of the functions of cultured EPCs (calculated as the ratio of the adhesive cells to all the cultured cells).

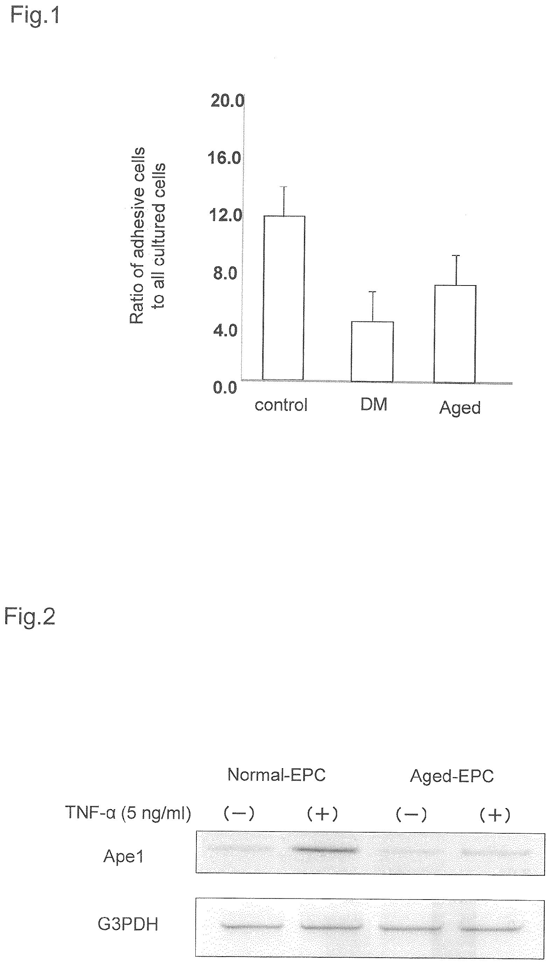

As shown in Figure 1, it was suggested that EPCs derived from the diabetic (DM) and aged (Aged) mice had the low ability to adhere to fibronectin and further had the reduced ability to form a colony, compared with normal mice (12 to 14 weeks old). Specifically, the cells that have adhered thereto forms a colony after growth, but the degree of colony formation (the number of cells per colony) is small. This can also be evaluated as the reduced ability to grow.

### 2. Ability of aged mouse-derived EPC to express Ape1 induced by inflammatory cytokine

EPCs were prepared in the same way as in the preceding paragraph from 12- to 14-week-old mice and an aged mouse (1 year and 6 months old). The prepared cells were cultured in the same way as in the preceding paragraph except that the culture period involved additional one week and the resulting confluent cells were collected 16 hours after medium replacement by a medium containing 5-10 ng/ml of the inflammatory cytokine TNF-α. RNAs were purified from the collected cells, and mRNA levels were measured by RT-PCR. The sequences of the primers used are as shown below.
Forward: atg ccg aag cgt ggg aaa aag (SEQ ID NO: 4)
Reverse: cag tgc tag gta tag ggt g (SEQ ID NO: 5)

As a result, EPCs derived from the aged mouse had the significantly reduced expression level of the Ape1 gene, compared with EPCs derived from the 12- to 14-week-old mice (Figure 2), demonstrating that the cells were also low responsive to the induction of expression by TNF-α. Usually, the expression level of Ape1 in cultured EPCs does not largely differ between aged and young mice. These results demonstrated that response to the induction of expression by TNF-α was significantly reduced in aged mouse-derived EPCs, compared with normal EPCs.

### 3. Introduction of Ape1 gene of EPC

Mouse endothelial progenitor cells were transfected with the human Ape1 gene using adenovirus. An Ape1 expression level (red) was measured in the EPCs thus transfected. The recombinant adenovirus harboring APE1 gene was introduced by two types of methods: a magnet transfection method using a magnet infection kit (Magnetofection(TM)-AdenoMag) and a standard method. Specifically, 20 µl of the Ape1-introduced virus (AdenoApe) was mixed with 2 µl of AdenoMag and incubated at room temperature for 20 minutes. Then, the resulting mixture was added to a culture plate containing subconfluent EPCs. This plate was placed on a magnetic plate and left standing for 60 minutes. Then, the cells were infected with the virus by usual culture at 37°C. The infected cells were immunologically reacted with anti-Ape1 antibodies and visualized (red) with TRITC secondary antibodies.

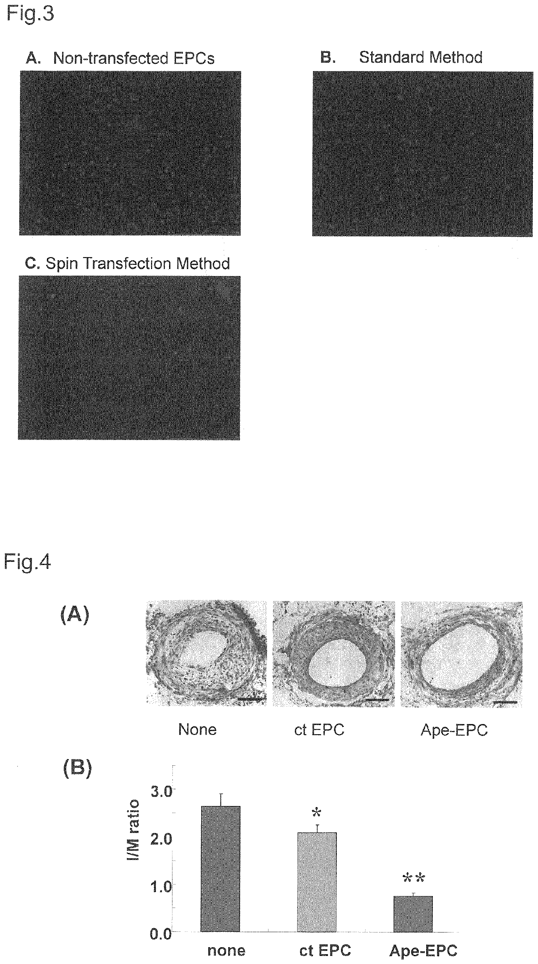

The results are shown in Figure 3. The results demonstrated that the magnet transfection method achieved efficient transection with the Ape1 gene.

### 4. Curing of vascular injury by Ape1-transfected EPC

The Ape1-transfected EPCs prepared in the preceding paragraph were introduced through the veins of 12- to 14-week-old mouse models with femoral arterial injury. Four weeks later, vascular remodeling (degree of neointimal thickening = I/M ratio) was evaluated.

The internal side of the femoral artery of each mouse was injured by the insertion of a wire (Cook Group Inc.). Then, the prepared EPCs (1 x 10⁴ cells/mouse) were introduced from the tail vein. Four weeks after the vascular injury, the femoral artery was excised and fixed, and a short-axis slice of the blood vessel was stained with HE for observation (Figure 4(A)). The areas of media (M) and thickened intima (I) were measured to calculate the degree of neointimal thickening (I/M ratio) (Figure 4(B)).

The Ape1-transfected EPC (Ape-EPC) group was confirmed to have the effect of highly improving the degree of neointimal thickening, although the normal EPC (ct EPC) group also exhibited the reduced degree of neointimal thickening, compared with a non-cell-introduced group (None).

### Example 2: Mesenchymal stem cell (MSC)

### 1. Establishment and preparation of mesenchymal stem cell line

The mesenchymal stem cell line was established by isolating NG2-positive cells from the peripheral capillary tissue-derived cells of a transgenic mouse expressing temperature-sensitive SV40T antigens (purchased from FACT Inc.) by the magnet sorting method using anti-NG2 antibodies.

The established cell line expressed gene populations such as NG2, CD146, PDGFR, and CD90 characteristic of pericytes and mesenchymal stem cells and retained the ability to differentiate into vascular cells, fat cells (expressing specific FABP4; identified with lipid droplets (oil red staining)), or osteoblasts (expressing osteopontin; identified with Ca deposition (Alizarin Red staining)). This cell line could be subcultured at 33°C in a collagen-coated culture dish containing DMEM (with 10% FCS) to maintain the immortality of the cells. For assay, the cells were cultured at 37°C to cause the inactivation of the SV40T antigens expressed in the cells. The resulting cells were used in the following experiment.

### 2. Evaluation of cell activity (oxidative stress-induced cell injury)

The mesenchymal stem cells thus cultured in a collagen-coated culture dish were infected with recombinant adenovirus for human Ape1 (hApe1) expression to force the mesenchymal stem cells to intracellularly express human Ape1 (hApe1). Cells infected with LacZ-expressing adenovirus were used as a control (Ct).

These cells were cultured under the culture conditions described above until the cells became subconfluent. Then, the medium was replaced by each of media containing various concentrations of hydrogen peroxide (H₂O₂), followed by culture for 24 hours to cause the oxidative stress-induced injury of the cells. The degree of the injury was evaluated by WST assay. The WST assay was conducted using WST-1 (Roche Applied Science) according to the instruction manual. The cell viability was calculated with that in an H₂O₂-untreated group as 100% and that in an SDS-killed cell group as 0%.

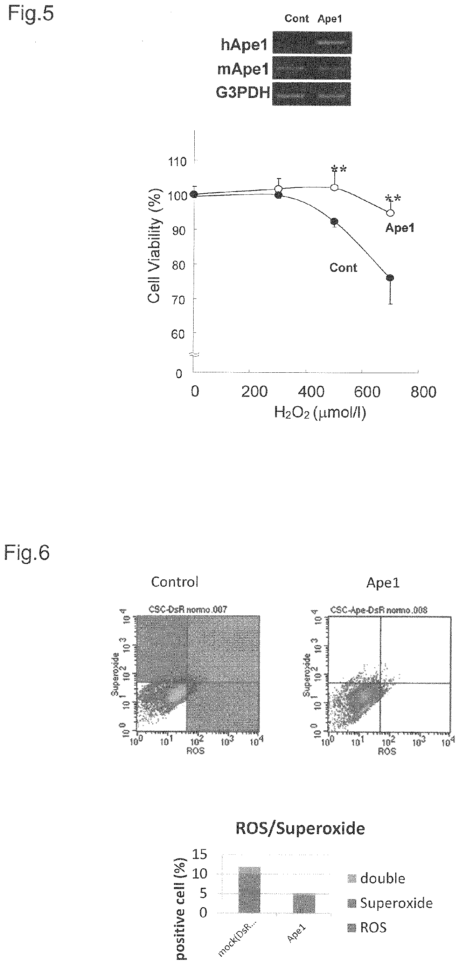

As shown in Figure 5, cell injury was observed from the level of 500 µM H₂O₂ in the control, and approximately 30% of the cells were injured in the presence of 700 µM H₂O₂. By contrast, no significant cell injury was observed in the Ape1-expressing mesenchymal stem cells in the presence of H₂O₂ in the same amounts as above. 50% or more of the cells were injured in both groups in the presence of a non-physiological concentration (1000 µM) of H₂O₂, showing no difference between the groups.

### Example 3: Myocardial stem cell

The human heart-derived myocardial stem cell line (CSC03) was established by enzymatic degradation method and subculture after bFGF addition from the microstructure of cardiac muscles collected from the right ventricle of a patient with chronic myocardial infarction.

Human Ape1-cDNA was inserted to the retrovirus vector pRetro-IRES-DsRed. CSC03 was transfected with pRetro-Ape-IRES-DsRed expressing Ape1 together with the fluorescent protein DsRed under the control of IRES. DsRed-positive cells were purified by FACS to selectively purify Ape1 gene-transfected cells. DsRed-positive cells were prepared as a control by transfection with pRetro-IRES-DsRed.

The Ape1-DsRed-CSC03 and DsRed-CSC03 cells were analyzed by FACS for their intracellular expression of reactive oxygen species (total ROS) and typical ROS superoxide.

As shown in Figure 6, the amounts of ROS and superoxide influencing the activity of CSC03 were confirmed to be 11.3% expression (production) of ROS (H₂O₂, ONOO-, HO·, NO, and ROO·) and superoxide (O₂·) in the control cells (DsRed-CSC03). By contrast, Ape1 gene-transfected CSC (Ape1: Ape1-DsRed-CSC03) reduced the production of ROS and superoxide to 5.1%.

### Industrial Applicability

The present invention can enhance the functions of stem cells/progenitor cells such as an angiogenic action, a vascular injury repairing action (re-endothelialization action), and oxidative stress resistance and achieve more effective revascularization therapy. The present disclosure can further achieve convenient evaluating function of stem cells/progenitor cells with Ape1 expression as an indicator, thereby realizing preoperative evaluation for cell-based revascularization therapy or the diagnosis of angiopathic disease.

### Free Text of Sequence Listing

SEQ ID NO: 1: Primer for Ape1 amplification (forward; for mice and rats)
SEQ ID NO: 2: Primer for Ape1 amplification (forward; for humans)
SEQ ID NO: 3: Primer for Ape1 amplification (reverse; for humans, mice and rats)
SEQ ID NO: 4: Primer for Ape1 RT-PCR (forward; for mice)
SEQ ID NO: 5: Primer for Ape1 RT-PCR (reverse; for mice)

## Claims

1. A cell preparation for use in a method of treatment selected from revascularization, organ regeneration, prevention or treatment of cancer, or prevention or treatment of ischemic diseases including lower limb ischemia, myocardial infarction, and cerebral infarction, the cell preparation comprising an endothelial progenitor cell or a mesenchymal stem cell wherein said method comprises a step of increasing Ape1 expression in said cells such that the cells have an improved ability to regenerate or generate an organ, or an improved revascularization or angiogenic capacity.

2. The cell preparation for use in a method according to claim 1, wherein increased expression of Ape1 is based on induction of Ape1 expression.

3. The cell preparation for use in a method according to claim 2, wherein the induction of Ape1 expression is based on introduction of an Ape1 gene.

4. The cell preparation for use in a method according to claim 2, wherein the induction of Ape1 expression is based on introduction of an Ape1 protein.

5. The cell preparation for use in a method according to claim 2, wherein the induction of Ape1 expression is based on an Ape1 expression inducer.

6. The cell preparation for use in a method according to claim 5, wherein the Ape1 expression inducer is one or more selected from TNF-α, IL-1β, and IF-γ.

7. The cell preparation for use in a method according to any one of claims 1 to 6, wherein the cell is derived from a patient in need of treatment using the cell preparation.

8. The cell preparation for use in a method according to any one of claims 1 to 7, wherein the cell preparation is intravenously administered, intramuscularly administered, or applied directly to a tissue.

9. The cell preparation for use in a method according to any one of claims 1 to 8, wherein the cell preparation comprises an endothelial progenitor cell.

10. The cell preparation for use in a method according to any one of claims 1 to 9, wherein the cell preparation comprises a mesenchymal stem cell.

11. The cell preparation for use in a method according to any one of claims 1 to 10, wherein the cell preparation is in a sheet form.

12. An *in vitro* method for improving the ability of an endothelial progenitor cell or mesenchymal stem cell to regenerate or generate an organ, comprising increasing expression of apurinic/apyrimidinic endonuclease 1 (Ape1) in the cell.

13. An *in vitro* method for improving the revascularization or angiogenic capacity of an endothelial progenitor cell or mesenchymal stem cell, comprising increasing expression of apurinic/apyrimidinic endonuclease 1 (Ape1) in the cell.

## Patentansprüche

1. Zellzubereitung zur Verwendung in einem Behandlungsverfahren, ausgewählt aus Revaskularisation, Organregeneration, Vorbeugung oder Behandlung von Krebs oder Vorbeugung oder Behandlung von ischämischen Erkrankungen, einschließlich Ischämie der unteren Extremitäten, Myokardinfarkt und Hirninfarkt, wobei die Zellzubereitung eine endotheliale Vorläuferzelle oder eine mesenchymale Stammzelle umfasst, wobei das Verfahren einen Schritt des Erhöhens der Ape1-Expression in den Zellen umfasst, sodass die Zellen eine verbesserte Fähigkeit zur Regeneration oder Bildung eines Organs oder eine verbesserte Revaskularisation oder angiogene Leistungsfähigkeit aufweisen.

2. Zellzubereitung zur Verwendung in einem Verfahren gemäß Anspruch 1, wobei die erhöhte Ape1-Expression auf der Induktion der Ape1-Expression basiert.

3. Zellzubereitung zur Verwendung in einem Verfahren gemäß Anspruch 2, wobei die Induktion der Ape1-Expression auf dem Einbringen eines Ape1-Gens basiert.

4. Zellzubereitung zur Verwendung in einem Verfahren gemäß Anspruch 2, wobei die Induktion der Ape1-Expression auf dem Einbringen eines Ape1-Proteins basiert.

5. Zellzubereitung zur Verwendung in einem Verfahren gemäß Anspruch 2, wobei die Induktion der Ape1-Expression auf einem Induktor der Ape1-Expression basiert.

6. Zellzubereitung zur Verwendung in einem Verfahren gemäß Anspruch 5, wobei der Induktor der Ape1-Expression einer oder mehr, ausgewählt aus TNF-α, IL-1 und IF-γ, ist.

7. Zellzubereitung zur Verwendung in einem Verfahren gemäß einem der Ansprüche 1 bis 6, wobei die Zelle aus einem Patienten, der einer Behandlung unter Verwendung der Zellzubereitung bedarf, stammt.

8. Zellzubereitung zur Verwendung in einem Verfahren gemäß einem der Ansprüche 1 bis 7, wobei die Zellzubereitung intravenös verabreicht wird, intramuskulär verabreicht wird oder direkt in ein Gewebe appliziert wird.

9. Zellzubereitung zur Verwendung in einem Verfahren gemäß einem der Ansprüche 1 bis 8, wobei die Zellzubereitung eine endotheliale Vorläuferzelle umfasst.

10. Zellzubereitung zur Verwendung in einem Verfahren gemäß einem der Ansprüche 1 bis 9, wobei die Zellzubereitung eine mesenchymale Stammzelle umfasst.

11. Zellzubereitung zur Verwendung in einem Verfahren gemäß einem der Ansprüche 1 bis 10, wobei die Zellzubereitung in Form einer Schicht (sheet) vorliegt.

12. In vitro-Verfahren zum Verbessern der Fähigkeit einer endothelialen Vorläuferzelle oder mesenchymalen Stammzelle zur Regeneration oder Bildung eines Organs, umfassend das Erhöhen der Expression der apurinischen/apyrimidinischen Endonuklease 1 (Ape1) in der Zelle.

13. In vitro-Verfahren zum Verbessern der Revaskularisation oder angiogenen Leistungsfähigkeit einer endothelialen Vorläuferzelle oder mesenchymalen Stammzelle, umfassend das Erhöhen der Expression der apurinischen/apyrimidinischen Endonuklease 1 (Ape1) in der Zelle.

## Revendications

1. Préparation cellulaire pour une utilisation dans une méthode de traitement sélectionnée parmi la revascularisation, la régénération d'organe, la prévention ou le traitement du cancer, ou la prévention ou le traitement de maladies ischémiques, incluant l'ischémie des membres inférieurs, l'infarctus du myocarde, et l'infarctus cérébral, la préparation cellulaire comprenant une cellule progénitrice endothéliale ou une cellule souche mésenchymateuse, dans laquelle ladite méthode comprend une étape d'augmentation de l'expression d'Ape1 dans lesdites cellules de sorte que les cellules ont une capacité améliorée pour régénérer ou générer un organe, ou une capacité améliorée de revascularisation ou d'angiogenèse.

2. Préparation cellulaire pour une utilisation dans une méthode selon la revendication 1, dans laquelle l'expression augmentée d'Ape1 est basée sur une induction de l'expression d'Ape1.

3. Préparation cellulaire pour une utilisation dans une méthode selon la revendication 2, dans laquelle l'induction de l'expression d'Ape1 est basée sur l'introduction d'un gène Ape1.

4. Préparation cellulaire pour une utilisation dans une méthode selon la revendication 2, dans laquelle l'induction de l'expression d'Ape1 est basée sur l'introduction d'une protéine Ape1.

5. Préparation cellulaire pour une utilisation dans une méthode selon la revendication 2, dans laquelle l'induction de l'expression d'Ape1 est basée sur un inducteur d'expression d'Ape1.

6. Préparation cellulaire pour une utilisation dans une méthode selon la revendication 5, dans laquelle l'inducteur d'expression d'Ape1 est un ou plusieurs sélectionné parmi TNF-α, IL-1β, et IF-γ.

7. Préparation cellulaire pour une utilisation dans une méthode selon l'une quelconque des revendications 1 à 6, dans laquelle la cellule est dérivée d'un patient nécessitant un traitement utilisant la préparation cellulaire.

8. Préparation cellulaire pour une utilisation dans une méthode selon l'une quelconque des revendications 1 à 7, dans laquelle la préparation cellulaire est administrée par voie intraveineuse, administrée par voie intramusculaire, ou appliquée directement à un tissu.

9. Préparation cellulaire pour une utilisation dans une méthode selon l'une quelconque des revendications 1 à 8, dans laquelle la préparation cellulaire comprend une cellule progénitrice endothéliale.

10. Préparation cellulaire pour une utilisation dans une méthode selon l'une quelconque des revendications 1 à 9, dans laquelle la préparation cellulaire comprend une cellule souche mésenchymateuse.

11. Préparation cellulaire pour une utilisation dans une méthode selon l'une quelconque des revendications 1 à 10, dans laquelle la préparation cellulaire est sous une forme de feuille.

12. Méthode *in vitro* pour améliorer la capacité d'une cellule progénitrice endothéliale ou d'une cellule souche mésenchymateuse à regénérer ou générer un organe, comprenant l'augmentation de l'expression de l'endonucléase apurinique/apyrimidinique 1 (Ape1) dans la cellule.

13. Méthode *in vitro* pour améliorer la capacité de revascularisation ou d'angiogenèse d'une cellule progénitrice endothéliale ou d'une cellule souche mésenchymateuse, comprenant l'augmentation de l'expression de l'endonucléase apurinique/apyrimidinique 1 (Ape1) dans la cellule.
